# EUROPEAN PATENT APPLICATION

(11) **EP 1 231 215 A1**
(43) Date of publication of application: **14.08.2002**
(21) Application number: 00961237.5
(22) Date of filing: 26.09.2000
(51) Int. Cl.: C07K 5/075, C07C 47/11, A23L 1/236

(54) **PROCESS FOR THE PRODUCTION OF ASPARTYLDIPEPTIDE ESTER DERIVATIVES, NOVEL INTERMEDIATES THEREFOR AND PROCESS FOR THE PRODUCTION OF THE INTERMEDIATES**

(30) Priority: 07.10.1999 JP 28739899; 27.12.1999 JP 37128499
(71) Applicant: Ajinomoto Co., Inc., Tokyo 104-8315 (JP)
(72) Inventor: NAGASHIMA, Kazutaka Amino Science Laboratories, Kawasaki-shi Kanagawa-ken 210-0801 (JP); AOKI, Yuuichi Amino Science Laboratories, Kawasaki-shi Kanagawa-ken 210-0801 (JP); TAKEMOTO, Tadashi Amino Science Laboratories, Kawasaki-shi Kanagawa-ken 210-0801 (JP); AMINO, Yusuke Amino Science Laboratories, Kawasaki-shi Kanagawa-ken 210-0801 (JP); FUNAKOSHI, Nao Amino Science Laboratories, Kawasaki-shi Kanagawa-ken 210-0801 (JP); ONO, Eriko Amino Science Laboratories, Kawasaki-shi Kanagawa-ken 210-0801 (JP)
(74) Representative: Nash, David Allan
(86) International application number: PCT/JP00/06626
(87) International publication number: WO 01/25260

(57) **Abstract**

In the present invention, efficient and industrial processes for production of aspartyl dipeptide ester derivative represented by the general formula (3) expected to serve as a sweetener, and aldehydes represented by the general formulae (1) and (2) which are intermediates therefor are provided.

Among them, a process for producing industrially and efficiently N-[N-[3-(3-hydroxy-4-methoxyphenyl) propyl]-L-α-aspartyl]-L-phenylalanine 1-methyl ester particularly excellent as a sweetener having a high sweetening potency, a development of intermediate for the production particularly useful and advantageous therefor, and an efficient process for production thereof are provided.

## Description

### Technical Field

The present invention relates to a novel process for production of an aspartyl dipeptide ester derivative expected to serve as a sweetener.

Further, the present invention relates to a process for production of a novel arylpropionaldehyde useful as an intermediate for the production of a N-[N-[3-(3-hydroxy-4-methoxyphenyl) propyl]-L- α-aspartyl]-L-phenylalanine 1-methyl ester important as a sweetener having a high sweetening potency among them, a process for production thereof, and a use thereof as an intermediate for the production thereof.

### Background Art

In recent years, as eating habits have been improved to a high level, fatness caused by excessive sugar intake and diseases accompanied by fatness have been at issue. Accordingly, the development of a low-calory sweetener (sweetening agent) that replaces sugar has been strongly in demand. As a sweetener that is widely used at present, there is aspartame which is excellent in safety and quality of sweetness, and however, is somewhat problematic in stability.

Under these background, the present inventors have found the compound represented by the following general formula (3) as a sweetener which is excellent in stability and moreover is better by far in a degree of sweetness, i.e., has an advantage in cost per a sweet taste. However, concerning an efficient process for production thereof, no method therefor has been known.

A N-[N-(3-phenylpropyl)-L-α-aspartyl]-L-phenylalanine 1-methyl ester and a N-[N-(3-methoxy-4-hydroxyphenylpropyl)-L-α-aspartyl]-L-phenylalanine 1-methyl ester which are poor in a degree of sweetness as compared to these compounds, are described in the International Patent Publication WO94/11391 However, in the publication, no example which should show a suitable operation for the synthesis thereof including a certain starting material employed was also contained, and concerning a process for production thereof, there was no mentions at all.

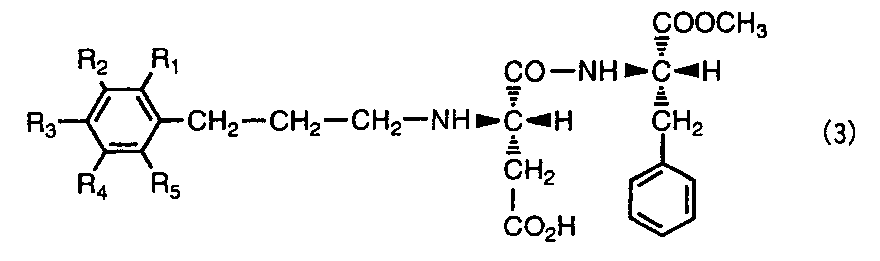

In the above formula, R₁, R₂, R₃, R₄ and R₅ are reciprocally independent and each denotes a substituent group selected from the group consisting of a hydrogen atom, a hydroxyl group, an alkoxy group having 1 to 3 carbon atoms, an alkyl group having 1 to 3 carbon atoms, and a hydroxyalkyloxy group having 2 or 3 carbon atoms, wherein two symbols of R₁ and R₂, or two symbols of R₂ and R₃ may be combined together to denote a methylenedioxy group.

Therefore, a development of an efficient and industrial process for production of an aspartyl dipeptide ester derivative represented by the general formula (3) described above, in particular, N-[N-[3-(3-hydroxy-4-methoxyphenyl) propyl]-L-α-aspartyl]-L-phenylalanine 1-methyl ester is requested.

### Problem to be solved by Invention

A problem to be solved by the present invention is to provide an efficient and industrial process for production of an aspartyl dipeptide ester derivative represented by the general formula (3) described above and expected to serve as a sweetener. In addition, a problem to be solved by the present invention is to provide an efficient and industrial process for production of an aldehyde represented by the following general formula (1) or (2), which is an intermediate compound for the aspartyl dipeptide ester derivative described above.

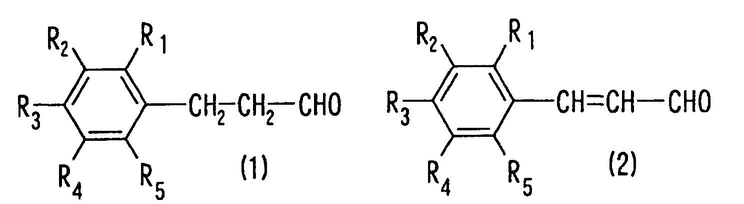

In the above formulae, R₁, R₂, R₃, R₄ and R₅ are reciprocally independent and each denotes a substituent group selected from the group consisting of a hydrogen atom, a hydroxyl group, an alkoxy group having 1 to 3 carbon atoms, an alkyl group having 1 to 3 carbon atoms, a benzyloxy group and a hydroxyalkyloxy group having 2 or 3 carbon atoms, wherein two symbols of R₁ and R₂, or two symbols of R₂ and R₃ may be combined together to denote a methylenedioxy group.

For example, concerning a production of 3-hydroxy-4-methoxyphenyl derivative desirable for the intermediate compound in the present invention, a process for firstly, alkylating reductively β -O-benzyl- α -L-aspartyl-L-phenylalanine methyl ester with 3-benzyloxy-4-methoxycinnamaldehyde and NaB(OAc)₃H, and next, removing the benzyl group of a protecting group was employed. However, 3-benzyloxy-4-methoxycinnamaldehyde used for the process is synthesized in the reaction which needs 4 reaction steps of so many steps from 3-hydroxy-4-methoxycinnamic acid as the starting material, as shown in the following reaction process 1, and therefore, it is hardly said that the compound is to be an industrially profitable ground substance for reaction. Further, it is hardly said that the process for production of the N-[N-[3-(3-hydroxy-4-methoxyphenyl) propyl]-L- α -aspartyl]-L-phenylalanine 1-methyl ester with the use of the aldehyde described above as the starting material is to be an industrially profitable process, because in addition to the reductive alkylation, a reaction for deprotection of a protecting group is additionally conducted necessarily.

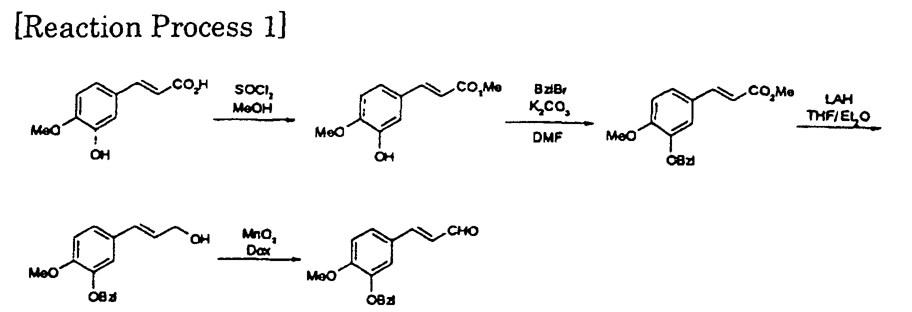

As stated above, the problem to be solved by the present invention is to provide an efficient and industrial process for production of the aspartyl dipeptide ester derivative represented by the general formula (3) described above and expected to serve as a sweetener, and also the aldehyde represented by the above general formula (1) or (2), an intermediate compound therefor. Also, it is to provide a process for producing industrially and efficiently the N-[N-[3-(3-hydroxy-4-methoxyphenyl) propyl]-L-α-aspartyl]-L-phenylalanine 1-methyl ester excellent particularly as a sweetener having a high sweetening potency, among them, a development of an intermediate for the production useful and profitable particularly therefor, and an efficient process for production thereof.

### Disclosure of Invention

The present inventors have earnestly studied on an efficient process for production of the compound represented by the above general formula (3), and as a result, found that the aspartyl dipeptide ester derivative as objective can be easily produced by alkylating reductively an aspartame in the presence of a catalyst, with the aldehyde represented by the above general formula (1) or general formula (2) and hydrogen, and reached a completion of the invention "a process for production of the aspartyl dipeptide ester derivative" involved in a typical one embodiment of the present invention.

In addition, the present inventors have found that the aldehyde represented by the above general formula (1) or general formula (2), is excellent as an intermediate for the production of the aspartyl dipeptide ester derivative in the present invention, and further found a process to produce industrially and efficiently these intermediate compounds.

That is to say, they have found that a hydrocinnamaldehyde derivative can be obtained in the process for selecting a cinnamic acid derivative as the starting material, reducing the carbon-carbon double bond therein selectively, preferably in the presence of the catalyst for hydrogenation (hydrogen addition) to obtain a hydrocinnamic acid derivative, and thereafter reducing the carboxyl group in the thus obtained derivative by half, preferably in the presence of the catalyst to conversion to a formyl group.

Further, they have found that a hydrocinnamaldehyde derivative can be obtained in the process for selecting a cinnamic acid derivative as the starting material, preferably reducing the carboxyl group therein by half (partially), preferably in the presence of the catalyst to conversion to a formyl group to obtain a cinnamaldehyde derivative, and thereafter reducing the carbon-carbon double bond in thus obtained derivative selectively, preferably in the presence of the catalyst for hydrogenation (hydrogen addition).

For example, as regards a N-[N-[3-(3-hydroxy-4-methoxyphenyl) propyl]-L- α -aspartyl]-L-phenylalanine 1-methyl ester excellent particularly as a sweetener having a high sweetening potency, 3-(3-hydroxy-4-methoxyphenyl) propionaldehyde may become an excellent intermediate for the production thereof, and thus, the compound can be produced industrially and efficiently based on the following reaction process 2.

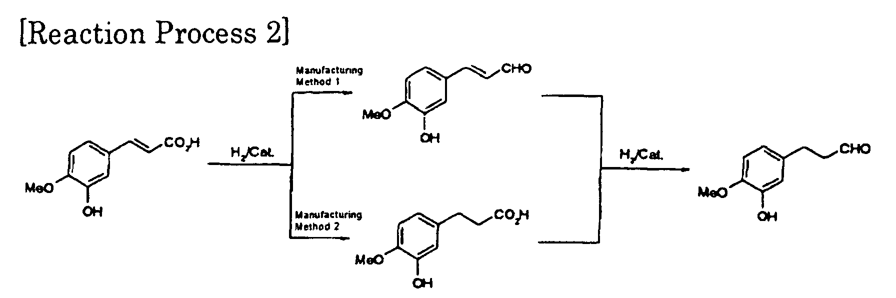

As stated above, the present invention includes, as the embodiments thereof, a process for production of the aspartyl dipeptide ester derivative, and a process for production of the cinnamaldehyde derivative or the hydrocinnamaldehyde derivative, and further includes a process for production of the N-[N-[3-(3-hydroxy-4-methoxyphenyl) propyl]-L- α -aspartyl]-L-phenylalanine 1-methyl ester excellent particularly as a sweetener having a high sweetening potency, processes for production of the cinnamaldehyde derivative and the hydrocinnamaldehyde derivative which are an intermediate for the production thereof, uses thereof, and the like. Concerning the various embodiments of the present invention, each embodiment thereof among them is explained in the followings.

### (Re.: Process for production of the aspartyl dipeptide ester derivative in the present invention)

The present invention on a process for production of the aspartyl dipeptide ester derivative is directed to the following process:

A process for producing an aspartyl dipeptide ester derivative represented by the following general formula (3), which comprises:
alkylating reductively an aspartame with an aldehyde represented by the following general formula (1) or formula (2), and hydrogen in the presence of a catalyst:

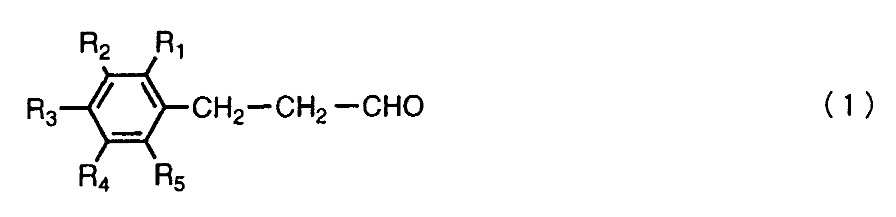

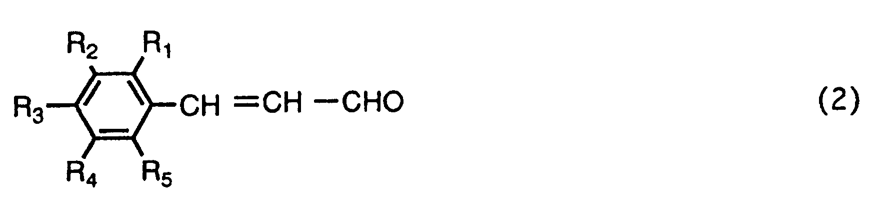

In the above formulae, R₁, R₂, R₃, R₄ and R₅ are reciprocally independent and each denotes a substituent group selected from the group consisting of a hydrogen atom, a hydroxyl group, an alkoxy group having 1 to 3 carbon atoms, an alkyl group having 1 to 3 carbon atoms, and a hydroxyalkyloxy group having 2 or 3 carbon atoms, wherein two symbols of R₁ and R₂, or two symbols of R₂ and R₃ may be combined together to denote a methylenedioxy group.

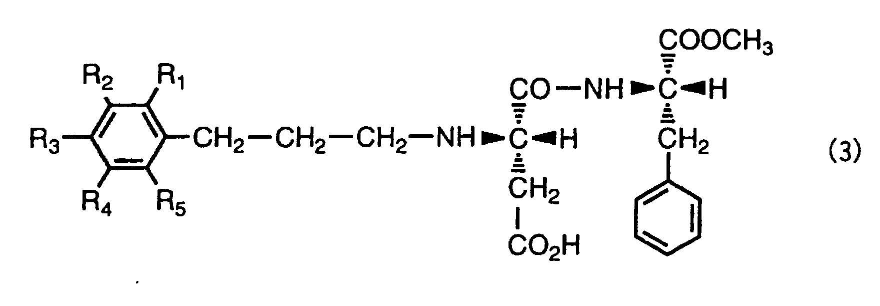

In the above formula, R₁, R₂, R₃, R₄ and R₅ are reciprocally independent and each denotes a substituent group selected from the group consisting of a hydrogen atom, a hydroxyl group, an alkoxy group having 1 to 3 carbon atoms, an alkyl group having 1 to 3 carbon atoms, and a hydroxyalkyloxy group having 2 or 3 carbon atoms, wherein two symbols of R₁ and R₂, or two symbols of R₂ and R₃ may be combined together to denote a methylenedioxy group.

In the aldehydes represented by the above formulae (1) and (2), for the reductive alkylation reaction in the present invention, in case that any one symbol of R₁ to R₅ has a hydroxyl group, the hydroxyl group may be protected with a benzyl group. That is to say, R₁ to R₅ are reciprocally independent and each may be a benzyloxy group. In this case, in the above reaction of the present invention, through removal formation of the benzyl group (benzyl group-removing reaction) in the benzyloxy moiety, the protected hydroxyl group may be converted into a hydroxyl group, and in the compound of formula (3) obtained, it does not have the benzyloxy group, and has a hydroxyl group instead.
(Re: Processes for production of the cinnamaldehyde derivative and the hydrocinnamaldehyde derivative in the present invention)
processes for production of the cinnamaldehyde derivative and the hydrocinnamaldehyde derivative in the present invention exist in the following contents.

That is to say, by reducing by half (partially) the carboxyl group in the cinnamic acid derivative represented by the following general formula (4), preferably in the presence of a catalyst, the carboxyl group therein is converted into a formyl group to obtain the cinnamaldehyde derivative represented by the above general formula (2). Subsequently, the carbon-carbon double bond therein is reduced selectively, preferably in the presence of a catalyst for hydrogenation, whereby the hydrocinnamaldehyde derivative represented by the above general formula (1) is obtained.

Further, in the cinnamic acid derivative represented by the following general formula (4), the carbon-carbon double bond therein is reduced selectively, preferably in the presence of a catalyst for hydrogenation to obtain the hydrocinnamic acid derivative represented by the following general formula (5). Subsequently, the carboxyl group therein is reduced by half (partially), preferably in the presence of a catalyst to convert the carboxyl group into a formyl group, whereby the hydrocinnamaldehyde derivative represented by the above general formula (1) is obtained.

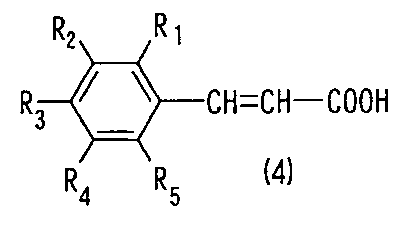

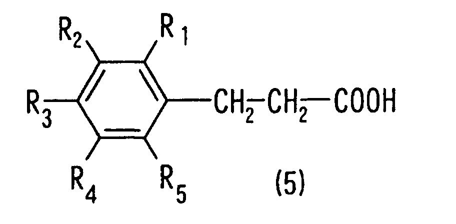

In the above formula, R₁, R₂, R₃, R₄ and R₅ are reciprocally independent and each denotes a substituent group selected from the group consisting of a hydrogen atom, a hydroxyl group, an alkoxy group having 1 to 3 carbon atoms, an alkyl group having 1 to 3 carbon atoms, and a hydroxyalkyloxy group having 2 or 3 carbon atoms, wherein two symbols of R₁ and R₂, or two symbols of R₂ and R₃ may be combined together to denote a methylenedioxy group.
(Re.: Novel hydrocinnamaldehyde derivative (arylpropionaldehyde), process for production thereof, and uses thereof in the present invention)

The present invention on the novel arylpropionaldehyde and the like, exists in the following contents.

### [1]

A process for producing a 3-(3-hydroxy-4-methoxyphenyl) propionaldehyde, which comprises:
a. subjecting a 3-hydoroxy-4-methoxycinnamaldehyde to a reaction for reducing a carbon-carbon double bond selectively; or
b. subjecting a 3-(3-hydoroxy-4-methoxyphenyl) propionic acid to a reaction for converting a carboxyl group into a formyl group,
to form (generate) a 3-(3-hydroxy-4-methoxyphenyl) propionaldehyde.

### [2]

In the above process [1], the process wherein said 3-hydoroxy-4-methoxycinnamaldehyde is obtained in the process for subjecting a 3-hydoroxy-4-methoxycinnamic acid to a reaction for converting a carboxyl group into a formyl group.

In the above processes [1] and [2], for the reaction for converting a carboxyl group into a formyl group, a reaction for reducing a carboxyl group by half (partially) to form a formyl group can be preferably employed.

### [3]

In the above process [1], the process wherein said 3-(3-hydoroxy-4-methoxyphenyl) propionic acid is obtained in the process for subjecting a 3-hydoroxy-4-methoxycinnamic acid to a reaction for reducing a carbon-carbon double bond selectively.

### [4]

In the above processes [1] and [3], the process wherein as for 3-hydoroxy-4-methoxycinnamaldehyde or 3-hydoroxy-4-methoxycinnamic acid, said reaction for reducing a carbon-carbon double bond selectively is conducted in the presence of a catalyst for hydrogenation, and preferably in the presence of a catalyst including at least one of palladium, platinum and rhodium based catalysts.

### [5]

In addition to the reaction in the above process, further a reductive alkylation reaction of the arylpropionaldehyde thus obtained with an aspartame is conducted, whereby a N-[N-[3-(3-hydroxy-4-methoxyphenyl) propyl]-L- α -aspartyl]-L-phenylalanine 1-methyl ester, an aspartame derivative is derived and produced. As a result, any above process comprising any reaction(s) in the above process(es) and said reductive alkylation reaction (a process for production of the above aspartame derivative) is contained in the present invention, as an embodiment thereof.

### [6]

A novel compound of 3-(3-hydroxy-4-methoxyphenyl) propionaldehyde.

### [7]

A novel compound of 3-(3-hydroxy-4-methoxyphenyl) propionaldehyde obtained in any one process of the above processes [1] to [4].

### Embodiments for carrying out Invention

Firstly, in terms of a process for production of the aspartyl dipeptide ester derivative in the present invention, it is explained in the followings.

In this process, in the solution which has dissolved or suspended aspartame, the aldehyde is dissolved, and a catalyst is added thereto, and thus obtained mixture is only stirred under an atmosphere of hydrogen gas, whereby the reaction proceeds. After completion of the reaction, the catalyst is removed by filtration, and the filtrate is concentrated to obtain the aspartyl dipeptide ester derivative as the crude product. Accordingly, the product is subjected to a purification with the chromatography or the like to be able to obtain the object compound of the aspartyl dipeptide ester derivative.

As for the reaction solvent employed, there is no particular limitations thereto, as far as a material inactive to a ground substance (substrate) for reaction, a catalyst and a product may be employed therefor.

A homogeneous organic solvent which can dissolve aspartame and the aldehyde, and which is a single solvent consisted of one kind of organic solvent only or a mixed solvent consisted of plural kinds of organic solvents, or a mixture of such organic solvent with water may be employed therefor.

For the organic solvent, for example, methanol, ethanol, tetrahydrofuran, acetonitrile and dimethylformamide are cited.

For the catalyst, a catalyst for hydrogenation, such as palladium based catalyst (palladium carbon and the like), platinum based catalyst (platinum carbon and the like), and rhodium based catalyst, and the like is preferably cited therefor.

The reductive alkylation reaction used in the present invention can be conducted through hydrogenation (hydrogen addition), and in such case, for the hydrogen pressure, preferably 0.1 to 1.0 MPa or so may be selected.

For the reaction temperature, the condition suitable for a reductive alkylation reaction can be selected. In order to suppress (limit) a secondary reaction and to promote the reaction desired (as objective), a temperature range of 15 to 50 °C or so, and a range for reaction time of 2 to 72 hours or so can be preferably selected.

As for a molar ratio of aspartame to the aldehyde as the starting materials, a range of preferably 0.5 to 1.5 moles or so of aspartame per 1 mole of the aldehyde can be used for reaction.

In the production of the aspartyl dipeptide ester derivative in the present invention (the reductive alkylation reaction), the typical processes are cited in the followings.

### [1]

The process for production of the aspartyl dipeptide ester derivative, wherein in the above formulae (1) to (3), R₂ is a hydroxyl group, R₃ is a methoxy group, and R₁, R₄ and R₅ are a hydrogen atom, and in the formulae (1) and (2), R₂ may be a benzyloxy group.

### [2]

The process for production of the aspartyl dipeptide ester derivative, wherein in the formulae (1) to (3), R₂ is a methyl group, R₃ is a hydroxyl group, and R₁, R₄ and R₅ are a hydrogen atom, and in the formulae (1) and (2), R₃ may be a benzyloxy group.

### [3]

The process for production of the aspartyl dipeptide ester derivative, wherein in the formulae (1) to (3), R₂ and R₃ are combined together to denote a methylenedioxy group, and R₁, R₄ and R₅ are a hydrogen atom.

### [4]

The process for production of the aspartyl dipeptide ester derivative, wherein in the formulae (1) to (3), R₁ is a hydroxyl group, R₃ is a methoxy group, and R₂, R₄ and R₅ are a hydrogen atom, and in the formulae (1) and (2), R₁ may be a benzyloxy group.

### [5]

The process for production of the aspartyl dipeptide ester derivative, wherein in the formulae (1) to (3), R₃ is a hydroxyl group, and R₁, R₂, R₄ and R₅ are a hydrogen atom, and in the formulae (1) and (2), R₃ may be a benzyloxy group.

### [6]

The process for production of the aspartyl dipeptide ester derivative, wherein in the formulae (1) to (3), R₂ is a hydroxyl group, R₃ is a methyl group, and R₁, R₄ and R₅ are a hydrogen atom, and in the formulae (1) and (2), R₂ may be a benzyloxy group.

### [7]

The process for production of the aspartyl dipeptide ester derivative, wherein in the formulae (1) to (3), R₁ and R₃ are a hydroxyl group, and R₂, R₄ and R₅ are a hydrogen atom, and in the formulae (1) and (2), R₁ and/or R₃ may be a benzyloxy group.

### [8]

The above process for production of the aspartyl dipeptide ester derivative, wherein said catalyst employed is at least one of palladium carbon and platinum carbon.

### [9]

The above process for production of the aspartyl dipeptide ester derivative, wherein a solvent is employed in the reaction for alkylating reductively said aspartame, and said solvent for the reaction is a methanol or a water-containing methanol.

### (Processes for production of the cinnamaldehyde derivative and the hydrocinnamaldehyde derivative in the present invention)

Next, as for the processes for production of the cinnamaldehyde derivative and the hydrocinnamaldehyde derivative in the present invention, they are explained in the followings in detail.

As shown in the manufacturing method 1 of the following scheme,
by reducing by half (partially) the carboxyl group in the cinnamic acid derivative represented by the general formula (4), preferably in the presence of a catalyst, the carboxyl group therein is converted into a formyl group to obtain the cinnamaldehyde derivative represented by the general formula (2).

Subsequently, the carbon-carbon double bond therein is reduced selectively, preferably in the presence of a catalyst for hydrogenation, whereby the hydrocinnamaldehyde derivative represented by the general formula (1) can be obtained.

Further, as shown in the manufacturing method 2 of the following scheme,
in the cinnamic acid derivative represented by the general formula (4), the carbon-carbon double bond therein is reduced selectively, preferably in the presence of a catalyst for hydrogenation to obtain the hydrocinnamic acid derivative represented by the general formula (5). Subsequently, the carboxyl group therein is reduced by half (partially), preferably in the presence of a catalyst to convert the carboxyl group into a formyl group, whereby the hydrocinnamaldehyde derivative represented by the general formula (1) can be obtained.

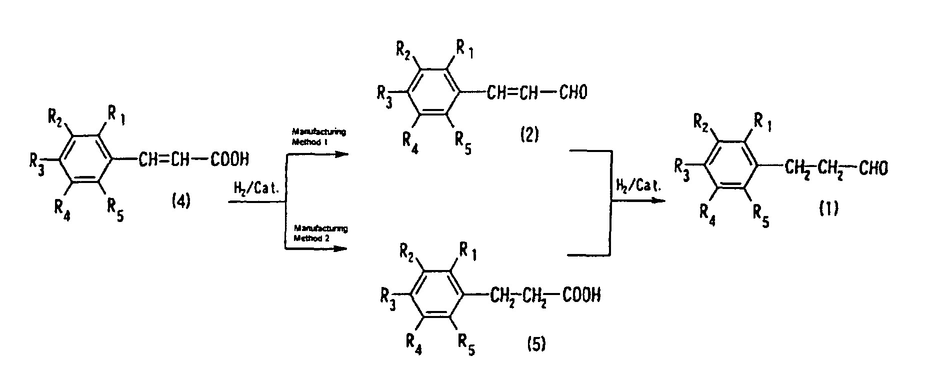

In the followings, on the basis of the above reaction process 2, with reference to a case of 3-hydroxy-4-methoxycinnamic acid desirable for the cinnamic acid derivative of the general formula (4) which is the starting material, as an example, the process for reaction is explained. However, also in case that the other cinnamic acid derivative is used for the starting material, the object product can be produced in the same manner, and the present invention is not limited to the sample, as long as a subject matter in the present invention does not come away (is not deviant).

Firstly, based on the manufacturing method 1 as an example it is explained.

In order to produce 3-hydroxy-4-methoxycinnamaldehyde from 3-hydroxy-4-methoxycinnamic acid, 3-hydroxy-4-methoxycinnamic acid may be directly reduced by half (partially). Preferably, it is directly reduced by half on the basis of the known process (Refer to Chem. Lett., 1998, 11, 1143.), whereby the starting material can be converted into the 3-hydroxy-4-methoxycinnamaldehyde as objective. This process comprises the step of reducing the 3-hydroxy-4-methoxycinnamic acid with hydrogen in an organic solvent in the presence of pivalic acid anhydride, palladium based compound such as palladium acetate, and triphenylphosphine derivative (triarylphosphine and the like) added.

As for the organic solvent employed, there is no particular limitations thereto, as far as a material inactive to a starting material for reaction, a catalyst and a product as objective may be employed therefor. For example, acetone, tetrahydrofuran, and the like are desirably employed.

As for a quantity consumed (employed) of pivalic acid anhydride, an equimolar (equimole) quantity or more of pivalic acid anhydride to the 3-hydroxy-4-methoxycinnamic acid may be used. For example, 1 to 5 times molar (moles) quantity or so of pivalic acid anhydride to the 3-hydroxy-4-methoxycinnamic acid may be used desirably.

As the triphenylphosphine derivative, triphenylphosphine, tritolylphosphine, and the like may be desirably employed. The palladium acetate or the like, and the triphenylphosphine derivative are employed as the catalyst, and therefore, for the quantity consumed thereof, only some moles % thereof is sufficient.

As for the reaction temperature, there is no particular limitations thereto. At a higher temperature, the reaction may be promoted, and can be finished (completed) in a shorter time.

In order to produce the 3-(3-hydroxy-4-methoxyphenyl) propionaldehyde from the 3-hydroxy-4-methoxycinnamaldehyde obtained in the above reaction, the carbon-carbon double bond in the 3-hydroxy-4-methoxycinnamaldehyde may be reduced selectively, preferably in the presence of the catalyst for hydrogenation. More preferably, the carbon-carbon double bond thereof is reduced selectively with reference to the known methods or the methods similar thereto (Refer to Engelhard. Ind. Tech. Bull., 1963, 4, 49.) for reducing a carbon-carbon double bond selectively, whereby the starting material can be converted into the 3-(3-hydroxy-4-methoxyphenyl) propionaldehyde. This process comprises the step of reducing selectively the 3-hydroxy-4-methoxycinnamaldehyde, in particular the carbon-carbon double bond therein with hydrogen in an organic solvent, for example, in the presence of the palladium based catalyst such as palladium-alumina, palladium-calcium carbonate and the like, the platinum based catalyst such as platinum carbon and the like, the rhodium based catalyst such as rhodium-alumina and the like, or the nickel based catalyst such as nickel-formic acid and the like.

As for the organic solvent employed, there is no particular limitations thereto. The organic solvent which can dissolve sufficiently the 3-hydroxy-4-methoxycinnamaldehyde of the starting material and the 3-(3-hydroxy-4-methoxyphenyl) propionaldehyde of the object product, and moreover which is inactive to the starting material for reaction, the catalyst and the object product may be employed therefor.

For the organic solvent, for example, a lower alcohol such as methanol, ethanol and the like, tetrahydrofuran, and the like can be used. More preferably, a lower alcohol may be employed, and further methanol may be employed preferably in view of cost.

For the catalyst employed, a generic and conventional catalyst for hydrogenation, such as the palladium based catalyst (palladium-alumina, palladium-calcium carbonate and the like), the platinum based catalyst (platinum carbon and the like), and the rhodium based catalyst (rhodium-alumina and the like), or the nickel based catalyst (nickel-formic acid and the like), can be used.

As for an amount consumed of the catalyst employed in such case, there is no particular limitations thereto. For example, in case of 5 % palladium-alumina, it may be preferably employed within a range of weight ratio of one hundredth (1/100) to one third (1/3) or so to the 3-hydroxy-4-methoxycinnamaldehyde.

This reaction, as stated above, can be conducted through hydrogenation (hydrogen addition), and at a pressure of 0.1 MPa, may proceed sufficiently. Also, under a hydrogen pressure of 0.1 to 5.0 MPa or so, the reaction can be conducted.

As for the reaction temperature, at an ordinary temperature or so, preferably 10 to 30°C or so, the reaction may proceed easily.

In order to improve a solubility of the starting material or the object product, and further to improve a reaction speed, the reaction solution may be heated up to 60 °C or so, and then the reaction can also be conducted at that temperature. Preferably, a temperature range of 20 to 50 °C or so can be selected.

As for the time for reaction, there is no particular limitations thereto. The reaction may be conducted within a range for the reaction time of 2 to 48 hours or so.

Next, based on the manufacturing method 2 as an example it is explained.

In order to produce the 3-(3-hydroxy-4-methoxyphenyl) propionic acid from the 3-hydroxy-4-methoxycinnamic acid, 3-hydroxy-4-methoxycinnamic acid, particularly the carbon-carbon double bond therein may be reduced selectively. Preferably, the carbon-carbon double bond thereof is reduced selectively on the basis of the known process or the process similar thereto (Refer to J. Org. Chem., 1994, 59, 2304.) for reducing a carbon-carbon double bond selectively, whereby the starting material can be converted into the 3-(3-hydroxy-4-methoxyphenyl) propionic acid as objective.

This process comprises the step of reducing selectively the 3-hydroxy-4-methoxycinnamic acid, in particular the carbon-carbon double bond therein with hydrogen in an organic solvent, for example, in the presence of the palladium based catalyst such as palladium carbon, palladium-alumina and the like, the platinum based catalyst such as platinum carbon and the like, the rhodium based catalyst such as rhodium-alumina and the like, or the nickel based catalyst such as Raney Nickel and the like.

As for the organic solvent employed, there is no particular limitations thereto. The organic solvent which can dissolve sufficiently the 3-hydroxy-4-methoxycinnamic acid of the starting material and the 3-(3-hydroxy-4-methoxyphenyl) propionic acid of the object product, and moreover which is inactive to the starting material for reaction, the catalyst and the object product may be employed therefor.

For the organic solvent, for example, a lower alcohol such as methanol, ethanol and the like, tetrahydrofuran, and a mixed solvent of such organic solvent(s) with water, and the like can be used. More preferably, a lower alcohol may be employed, and further methanol may be employed preferably in view of cost. Further more preferably, a mixed solvent of methanol and water may be employed.

As the catalyst, if employed, as stated above, a generic and conventional catalyst for hydrogenation, such as the palladium based catalyst (palladium carbon, palladium-alumina and the like), the platinum based catalyst (platinum carbon and the like), and the rhodium based catalyst (rhodium-alumina and the like), or the nickel based catalyst (Raney Nickel and the like), can be used.

As for an amount consumed of the catalyst, there is no particular limitations thereto. For example, in case of 10 % palladium carbon in the water content of 50 %, it may be preferably employed within a range of weight ratio of one hundredth (1/100) to one third (1/3) or so to the 3-hydroxy-4-methoxycinnamic acid.

This reaction, as stated above, can be conducted through hydrogenation (hydrogen addition), and at a pressure of 0.1 MPa, may proceed sufficiently. Also, under a hydrogen pressure of 0.1 to 1.0 MPa or so, the reaction can be conducted.

As for the reaction temperature, at an ordinary temperature or so, preferably 10 to 30°C or so, the reaction may proceed easily.

In order to improve a solubility of the starting material or the object product, and further to improve a reaction speed, the reaction solution may be heated up to 60 °C or so, and then the reaction can also be conducted at that temperature. Preferably, a temperature range of 30 to 50 °C or so can be selected.

As for the time for reaction, there is no particular limitations thereto. The reaction may be conducted within a range for the reaction time of 2 to 48 hours or so.

In order to produce a 3-(3-hydroxy-4-methoxyphenyl) propionaldehyde from the 3-(3-hydroxy-4-methoxyphenyl) propionic acid obtained in the above reaction, the 3-(3-hydroxy-4-methoxyphenyl) propionic acid may be directly reduced by half (partially). The process in such case may be conducted in the same manner as the case to the process for half reduction of the above 3-hydroxy-4-methoxycinnamic acid.

In order to produce an aspartyl dipeptide ester derivative, in particular preferably a N-[N-[3-(3-hydroxy-4-methoxyphenyl) propyl]-L- α -aspartyl]-L-phenylalanine 1-methyl ester, from the cinnamaldehyde derivative or the hydrocinnamaldehyde derivative thus obtained, in particular preferably form the 3-(3-hydroxy-4-methoxyphenyl) propionaldehyde, there is no particular difficulty. The process for the production may be conducted as explained in the process for production of the aspartyl dipeptide ester derivative, described above. Particularly, the aldehyde derivative obtained here, is alkylated reductively with an α -L-aspartyl-L-phenylalanine methyl ester (aspartame) under a condition for hydrogenation (hydrogen addition), and thereby the aspartyl dipeptide ester derivative can be easily produced. Specifically, in the solvent which can dissolve the starting materials, for example, the solvent, such as alcohol, water-containing alcohol or the like, in the presence of the catalyst for hydrogenation, for example, palladium based catalyst and the like, an alkylation reaction is reductively conducted with hydrogen, more preferably under a suitable or effective reaction temperature and pressure, and thereby the object compound described above can be produced.

From the reaction mixture obtained in the reaction, for example, after the catalyst, if used, is removed, the object compound of the aspartyl dipeptide ester derivative with high purity for a sweetener having a high sweetening potency, described above can be separated efficiently depending on the purification step(s), where necessary, such as a purification with a chromatography or the like. For example, in case of N-[N-[3-(3-hydroxy-4-methoxyphenyl) propyl]-L- a-aspartyl]-L-phenylalanine 1-methyl ester, for example, from the reaction mixture obtained in the reaction, after the catalyst is removed, the solvent is removed by distillation, and thus obtained residue may be subjected to a purification with a silica gel chromatography (for example, eluting solvent: ethyl acetate /methanol /chloroform = 3/3/2). Thus eluted fractions containing the object compound, are concentrated under reduced pressure to be able to obtain N-[N-[3-(3-hydroxy-4-methoxyphenyl) propyl]-L-α-aspartyl]-L-phenylalanine 1-methyl ester in the solid form.

### Examples

The present invention will be explained further in detail with reference to the following Examples therefor. However, the present invention is not limited by the following Examples, as far as its subject matter therein does not come off.

### (Example 1)

### Production of N-[N-[3-(3-hydroxy-4-methoxyphenyl) propyl]-L-α-aspartyl]-L-phenylalanine 1-methyl ester

Aspartame (5.89 g, 20.0 mmol) and 3-(3-hydroxy-4-methoxyphenyl) propionaldehyde (3.42 g, 19.0 mmol) were added to 80 % methanol aqueous solution (200 ml), and the mixture was stirred at 40 °C in a short time. To this solution, 10 % palladium carbon in the water content of 50 % (1.78 g) was added, and thus obtained mixture was stirred under hydrogen atmosphere of normal pressure (0.1 MPa) at 40 °C for 40 hours. The reaction solution was filtrated to remove the catalyst, and the filtrate was subjected to the high performance liquid chromatography (HPLC) for determination to produce the title compound (6.89 g, 15.0 mmol, 78.9 %).

### (Example 2)

### Production of N-[N-[3-(3-hydroxy-4-methoxyphenyl) propyl]-L-α-aspartyl]-L-phenylalanine 1-methyl ester

Aspartame (5.89 g, 20.0 mmol) and 3-(3-hydroxy-4-methoxyphenyl) propionaldehyde (3.42 g, 19.0 mmol) were added to methanol (150 ml), and the mixture was stirred in a short time. To thus obtained slurry, 10 % palladium carbon in the water content of 50 % (1.78 g) was added, and thus obtained mixture was stirred under hydrogen atmosphere of normal pressure (0.1 MPa) at room temperature for 24 hours. The reaction solution was filtrated to remove the catalyst, and the filtrate was subjected to the HPLC for determination to produce the title compound (5.92 g, 12.9 mmol, 67.9 %).

### (Example 3)

### Production of N-[N-[3-(2-hydroxy-4-methoxyphenyl) propyl]-L-α-aspartyl]-L-phenylalanine 1-methyl ester

Aspartame (5.89 g, 20.0 mmol) and 3-(2-hydroxy-4-methoxyphenyl) propionaldehyde (3.42 g, 19.0 mmol) were added to 80 % methanol aqueous solution (200 ml), and the mixture was stirred at 40 °C in a short time. To this solution, 10 % palladium carbon in the water content of 50 % (1.78 g) was added, and thus obtained mixture was stirred under hydrogen atmosphere of normal pressure (0.1 MPa) at 40 °C for 48 hours. The reaction solution was filtrated to remove the catalyst, and the filtrate was subjected to the HPLC for determination to generate the title compound (6.26 g, 13.7 mmol, 72.1%).

### (Example 4)

### Production of N-[N-[3-(3-hydroxy-4-methoxyphenyl) propyl]-L-α-aspartyl]-L-phenylalanine 1-methyl ester

Aspartame (0.40 g, 1.47 mmol) and 3-hydroxy-4-methoxycinnamaldehyde (0.25 g, 1.40 mmol) were added to methanol (13 ml), and the mixture was stirred in a short time. To thus obtained slurry, 10 % palladium carbon in the water content of 50 % (0.12 g) was added, and thus obtained mixture was stirred under hydrogen atmosphere of normal pressure (0.1 MPa) at room temperature for 24 hours. The reaction solution was filtrated to remove the catalyst, and the filtrate was subjected to the HPLC for determination to generate the title compound (0.21 g, 0.46 mmol, 32.8 %).

### (Example 5)

### Production of N-[N-[3-(3-methyl-4-hydroxyphenyl) propyl]-L-α-aspartyl]-L-phenylalanine 1-methyl ester

Aspartame (5.89 g, 20.0 mmol) and 3-(3-methyl-4-hydroxyphenyl) propionaldehyde (3.12 g, 19.0 mmol) were added to 60 % methanol aqueous solution (150 ml), and the mixture was stirred in a short time. To thus obtained slurry, 10 % palladium carbon in the water content of 50 % (2.00 g) was added, and thus obtained mixture was stirred under hydrogen atmosphere of normal pressure (0.1 MPa) at room temperature for 48 hours. The reaction solution was filtrated to remove the catalyst, and the filtrate was subjected to the HPLC for determination to generate the title compound (6.92 g, 15.7 mmol, 82.6 %).

### (Example 6)

### Production of N-[N-[3-(3,4-methylenedioxyphenyl) propyl]-L-α-aspartyl]-L-phenylalanine 1-methyl ester

Aspartame (5.89 g, 20.0 mmol) and 3-(3,4-methylenedioxyphenyl) propionaldehyde (3.12 g, 19.0 mmol) were added to 80 % methanol aqueous solution, and the mixture was stirred in a short time. To thus obtained slurry, 10 % palladium carbon in the water content of 50 % (1.78 g) was added, and thus obtained mixture was stirred under hydrogen atmosphere of normal pressure (0.1 MPa) at room temperature for 48 hours. The reaction solution was filtrated to remove the catalyst, and the filtrate was subjected to the HPLC (high performance liquid chromatography) for determination to generate the title compound (6.21 g, 14.0 mmol, 73.7 %).

### (Example 7)

### Production of N-[N-[3-(4-hydroxyphenyl) propyl]-L-α-aspartyl]-L-phenylalanine 1-methyl ester

Aspartame (5.89 g, 20.0 mmol) and 3-(4-hydroxyphenyl) propionaldehyde (2.85 g, 19.0 mmol) were added to 60 % methanol aqueous solution (200 ml), and the mixture was stirred at 40 °C in a short time. To this solution, 10 % palladium carbon in the water content of 50 % (1.78 g) was added, and thus obtained mixture was stirred under hydrogen atmosphere of normal pressure (0.1 MPa) at 40 °C for 48 hours. The reaction solution was filtrated to remove the catalyst, and the filtrate was subjected to the HPLC for determination to produce the title compound (6.18 g, 14.4 mmol, 75.8 %).

### (Example 8)

### Production of N-[N-[3-(3-hydroxy-4-methylphenyl) propyl]-L-α- aspartyll-L-phenylalanine 1-methyl ester

Aspartame (5.89 g, 20.0 mmol) and 3-(3-hydroxy-4-methylphenyl) propionaldehyde (3.12 g, 19.0 mmol) were added to 80 % methanol aqueous solution (150 ml), and the mixture was stirred in a short time. To thus obtained slurry, 10 % palladium carbon in the water content of 50 % (1.78 g) was added, and thus obtained mixture was stirred under hydrogen atmosphere of normal pressure (0.1 MPa) at room temperature for 48 hours. The reaction solution was filtrated to remove the catalyst, and the filtrate was subjected to the HPLC for determination to produce the title compound (6.97 g, 15.8 mmol, 83.2 %).

### (Example 9)

### Production of N-[N-[3-(2-hydroxy-3-methoxyphenyl) propyl]-L-α- aspartyl]-L-phenylalanine 1-methyl ester

Aspartame (5.89 g, 20.0 mmol) and 2-hydroxy-3-methoxycinnamaldehyde [3-(2-hydroxy-3-methoxyphenyl)-2-propenyl aldehyde] (3.38 g, 18.0 mmol) were added to 80 % methanol aqueous solution (200 ml), and the mixture was stirred in a short time. To thus obtained slurry, 10 % palladium carbon in the water content of 50 % (1.78 g) was added, and thus obtained mixture was stirred under hydrogen atmosphere of normal pressure (0.1 MPa) at room temperature for 48 hours. The reaction solution was filtrated to remove the catalyst, and the filtrate was subjected to the HPLC for determination to produce the title compound (4.26 g, 9.3 mmol, 48.9 %).

### (Example 10)

### Production of N-[N-[3-(3-hydroxy-4-methoxyphenyl) propyl]-L-α-aspartyl]-L-phenylalanine 1-methyl ester

Aspartame (5.89 g, 20.0 mmol) and 3-(3-benzyloxy-4-methoxyphenyl) propionaldehyde (5.15 g, 19.0 mmol) were added to 80 % methanol aqueous solution (200 ml), and the mixture was stirred in a short time. To thus obtained slurry, 10 % palladium carbon in the water content of 50 % was added, and thus obtained mixture was stirred under hydrogen atmosphere of normal pressure (0.1 MPa) at room temperature for 40 hours. The reaction solution was filtrated to remove the catalyst, and the filtrate was subjected to the HPLC for determination to produce the title compound (7.01 g, 15.3 mmol, 80.5 %).

### (Example 11)

### Production of N-[N-[3-(3-hydroxy-4-methoxylphenyl) propyl]-L-α-aspartyl]-L-phenylalanine 1-methyl ester

Aspartame (5.89 g, 20.0 mmol) and 3-(3-hydroxy-4-methoxyphenyl) propionaldehyde (3.42 g, 19.0 mmol) were added to 80 % methanol aqueous solution (180 ml), and further acetic acid (10 ml) was added thereto, and the mixture was stirred in a short time. To thus obtained slurry, 10 % palladium carbon in the water content of 50 % (1.78 g) was added, and thus obtained mixture was stirred under hydrogen atmosphere of normal pressure (0.1 MPa) at room temperature for 24 hours. The reaction solution was filtrated to remove the catalyst, and the filtrate was subjected to the HPLC for determination to produce the title compound (6.72 g, 14.7 mmol, 77.4 %).

### (Example 12)

### Production of N-[N-[3-(2,4-dihydroxyphenyl) propyl]-L-α-aspartyl]-L-phenylalanine 1-methyl ester

Aspartame (5.89 g, 20.0 mmol) and 3-(2,4-dihydroxyphenyl) propionaldehyde (3.15 g, 19.0 mmol) were added to 80 % methanol aqueous solution (200 ml), and the mixture was stirred in a short time. To thus obtained slurry, 10 % palladium carbon in the water content of 50 % was added, and thus obtained mixture was stirred under hydrogen atmosphere of normal pressure (0.1 MPa) at room temperature for 40 hours. The reaction solution was filtrated to remove the catalyst, and the filtrate was subjected to the HPLC for determination to produce the title compound (5.94 g, 13.4 mmol, 70.5 %).

### (Example 13)

### Production of 3-hydroxy-4-methoxycinnamaldehyde

In a chemical reactor for hydrogen addition (hydrogenation) under elevated pressure, previously tetrahydrofuran (64 ml) was bubbled with nitrogen gas for 10 minutes.

Palladium acetate (58 mg, 0.257 mmol), tri(p-tolyl) phosphine (157 mg, 0.515 mmol), 3-hydroxy-4-methoxycinnamic acid (5.00 g, 25.7 mmol) and pivalic acid anhydride (14.4 g, 77.2 mmol) were added thereto, and thereafter the mixture was bubbled with nitrogen gas for 30 minutes to substitute nitrogen gas completely for the gas in the system of reaction, whereby the system was filled with nitrogen gas. Next, hydrogen gas was added thereinto to substitute hydrogen gas for the gas in the system, and then the mixture was stirred under hydrogen pressure of 3.4 MPa at 80 °C for 24 hours for reaction. Thus obtained reaction solution was concentrated under reduced pressure to remove tetrahydrofuran by vaporization. The remaining residue was subjected to a purification process with a silica gel column chromatography (eluting solvent: toluene /ethyl acetate =4/1) to obtain 3-hydroxy-4-methoxycinnamaldehyde (2.26 g, 12.7 mmol, yield: 49 %).

### (Example 14)

### Production of 3-(3-hydroxy-4-methoxyphenyl) propionic acid

3-Hydroxy-4-methoxycinnamic acid (15.0 g, 77.2 mmo) and 10 % palladium carbon in the water content of 50 % (2.26 g) were added to a mixed solvent (330 ml) of methanol and water (mixing ratio of 10 : 1 v/v), and the mixture was stirred under hydrogen atmosphere of normal pressure (0.1 MPa) at 50 °C for 5 hours for reaction. The reaction solution was filtrated to remove the catalyst, and the filtrate was concentrated under reduced pressure to solidification to obtain 3-(3-hydroxy-4-methoxyphenyl) propionic acid (15.1 g, 76.7 mmol, yield: 99 %).

### (Example 15)

### Production of 3-(3-hydroxy-4-methoxyphenyl) propionaldehyde

3-Hydroxy-4-methoxycinnamaldehyde (8.66 g, 48.6 mmol) and 5 % palladium-alumina (aluminum oxide) (0.540 g) were added to methanol (144 ml), and the mixture was stirred under hydrogen atmosphere of normal pressure (0.1 MPa) at 35 °C for 7 hours for reaction. The reaction solution was filtrated to remove the catalyst, and further the catalyst was washed with methanol (40 ml). The filtrate and the wash solution were combined together, and then concentrated. Thus concentrated solution was subjected to a purification process with a silica gel column chromatography (eluting solvent: toluene /ethyl acetate =4/1). The thus eluted fractions containing the object compound were concentrated under reduced pressure to obtain crude 3-(3-hydroxy-4-methoxyphenyl) propionaldehyde (6.86 g, 38.1 mmol, yield: 78 %) in the slightly yellow coloured solid.

Thus obtained crude 3-(3-hydroxy-4-methoxyphenyl) propionaldehyde was recrystallized from toluene to obtain purified 3-(3-hydroxy-4-methoxyphenyl) propionaldehyde (5.83 g, 32.3 mmol, crystallization yield: 85 %) in the white crystalline form.

### (Example 16)

### Production of 3-(3-hydroxy-4-methoxyphenyl) propionaldehyde

Into a chemical reactor for hydrogen addition (hydrogenation) under elevated pressure, 3-(3-hydroxy-4-methoxyphenyl) propionic acid (5.09 g, 25.9 mmol), pivalic acid anhydride (14.3 g, 76.6 mmol), and tetrahydrofuran (64 ml) were added, and thereafter the mixture was bubbled with nitrogen gas for 10 minutes. Palladium acetate (57 mg, 0.254 mmol) and triphenylphosphine (349 mg, 1.33 mmol) were added thereto, and thereafter the mixture was bubbled with nitrogen gas for 20 minutes to substitute nitrogen gas completely for the gas in the system of reaction, whereby the system was filled with nitrogen gas. Next, hydrogen gas was added thereinto to substitute hydrogen gas for the gas in the system, and then the mixture was stirred under hydrogen pressure of 5.4 MPa at 80 °C for 24 hours for reaction. Thus obtained reaction solution was concentrated under reduced pressure to remove tetrahydrofuran by vaporization. The remaining residue was subjected to a purification process with a silica gel column chromatography (eluting solvent: hexane /ethyl acetate =2/1). The thus eluted fractions containing the object compound were concentrated under reduced pressure to obtain crude 3-(3-hydroxy-4-methoxyphenyl) propionaldehyde (2.26 g, 12.5 mmol, yield: 48 %) in the slightly yellow coloured solid.

Thus obtained crude 3-(3-hydroxy-4-methoxyphenyl) propionaldehyde was recrystallized from toluene to obtain purified 3-(3-hydroxy-4-methoxyphenyl) propionaldehyde (1.94 g, 10.8 mmol, crystallization yield: 86 %) in the white crystalline form.

### (Example 17)

### Physical properties on 3-(3-hydroxy-4-methoxyphenyl) propionaldehyde

The physical properties on the title compound obtained in the present invention were in the followings.

### White crystals

### (Differential thermal analysis)

Temperature range for the determination: 50-300 °C; Heating-up
speed: 10 °C/minute; Melting point: 71 °C.
¹H- NMR (CDCl₃) :
δ : 2.70-2.75 (m, 2H), 2.84-2.89 (m, 2H), 3.86 (s, 3H), 5.64 (s, 1H), 6.64-6.68 (m, 1H), 6.75-6.78 (m, 2H), 9.80 (t, J=1.5 Hz, 1H). ESI-MS:
Calculation: C₁₀H₁₂O₃=180.2, Analysis: 179.2(MH⁻).

### (Example 18)

### Production of N-[N-[3-(3-hydroxy-4-methoxyphenyl) propyl]-L-α-aspartyl]-L-phenylalanine 1-methyl ester

3-(3-Hydroxy-4-methoxyphenyl) propionaldehyde (1.50 g, 8.32 mmol) and aspartame (2.57 g, 8.74 mmol) were added to a mixed solvent (86 ml) of methanol and water (Mixing ratio of 4:1 v/v), and 10 % palladium carbon in the water content of 50 % (0.77 g) was added thereto. The mixture was stirred under hydrogen atmosphere of normal pressure (0.1 MPa) at 35 °C for 48 hours for reaction. After completion of the reaction, the catalyst was removed by filtration and further washed with methanol (20 ml). The filtrate and the wash solution were combined together, and subjected to the high performance liquid chromatography (HPLC) for determination to generate N-[N-[3-(3-hydroxy-4-methoxyphenyl) propyl]-L- α-aspartyl]-L-phenylalanine 1-methyl ester (2.69 g, 5.87 mmol, yield: 71 %).

Data on the NMR spectrum and mass spectrum of N-[N-[3-(3-hydroxy-4-methoxyphenyl) propyl]-L- α -aspartyl]-L-phenylalanine 1-methyl ester are shown in the followings.
¹H-NMR (DMSO-d₆):
δ: 1.50-1.60 (m, 2H), 2.15-2.40 (m, 6H), 2.87-2.97 (dd, 1H), 3.05-3.13 (dd, 1H), 3.37-3.43 (m, 1H), 3.62 (s, 3H), 3.71 (s, 3H), 4.50-4.60 (m, 1H), 6.52 (d, 1H), 6.60 (s, 1H), 6.79 (d, 1H), 7.18-7.30 (m, 5H), 8.52 (d, 1H), 8.80 (brs, 1H). ESI-MS:
Calculation: C₂₄H₃₀N₂O₇=458.5, Analysis: 459.2(MH⁺).

### Effect of Invention

According to the present invention, a N-[N-[3-(phenyl with substituent group(s)) propyl]-L- α -aspartyl]-L-phenylalanine 1-methyl ester expected to serve as a sweetener, can be produced industrially and efficiently.

Further, according to the present invention, a cinnamaldehyde derivative derivative and a hydrocinnamaldehyde derivative useful as intermediates for the production of the above N-[N-[3-(phenyl with substituent group(s)) propyl]-L-α-aspartyl]-L-phenylalanine 1-methyl ester, can be produced industrially and efficiently.

In addition, by using a 3-(3-hydroxy-4-methoxyphenyl) propionaldehyde which is a novel arylpropionaldehyde used desirably as an intermediate for the production in the present invention, a N-[N-[3-(3-hydroxy-4-methoxyphenyl) propyl]-L-α-aspartyl]-L-phenylalanine 1-methyl ester particularly useful as a sweetener having a high sweetening potency can be produced industrially and efficiently.

The above arylpropionaldehyde is a novel compound, and it can be produced easily and efficiently based on the process for using a 3-hydroxy-4-methoxycinnamic acid as the starting material,
converting the carboxyl group in the compound into a formyl group, and then reducing the carbon-carbon double bond therein selectively, or
reducing the carbon-carbon double bond in the compound selectively, and then converting the carboxyl group therein into a formyl group.

As explained above, according to the process in the present invention, a N-[N-[3-(3-hydroxy-4-methoxyphenyl) propyl]-L-α-aspartyl]-L-phenylalanine 1-methyl ester which is a particularly useful sweetener having a high sweetening potency, can be produced industrially and advantageously.

## Claims

1. A process for producing an aspartyl dipeptide ester derivative represented by the following general formula (3), which comprises:
alkylating reductively an aspartame with an aldehyde represented by the following general formulae (1) or (2), and 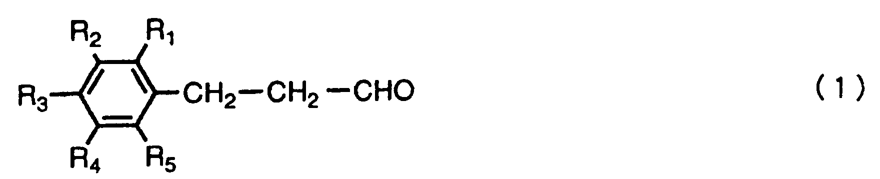 hydrogen in the presence of catalyst: 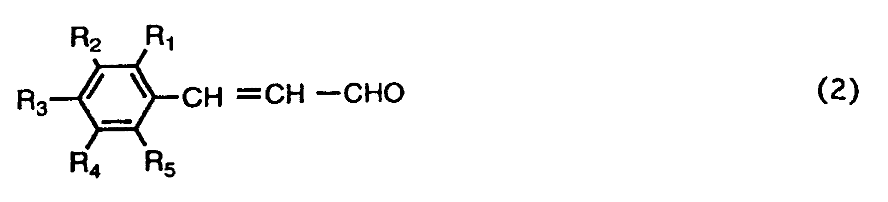 In the formulae (1) and (2), R₁, R₂, R₃, R₄ and R₅ are reciprocally independent and each denotes a substituent group selected from the group consisting of a hydrogen atom, a hydroxyl group, an alkoxy group having 1 to 3 carbon atoms, an alkyl group having 1 to 3 carbon atoms, a benzyloxy group and a hydroxyalkyloxy group having 2 or 3 carbon atoms, wherein two symbols of R₁ and R₂, or two symbols of R₂ and R₃ may be combined together to denote a methylenedioxy group. 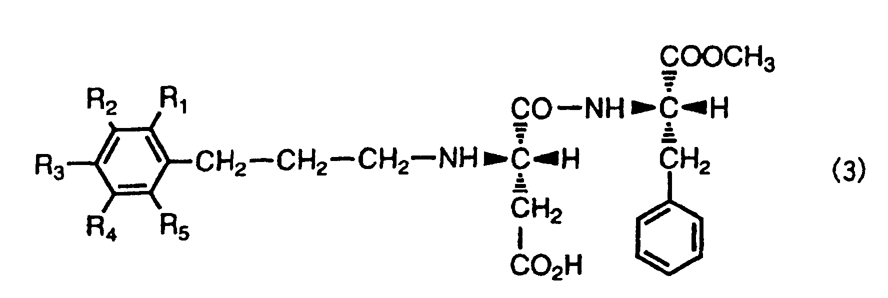 In the formula (3), R₁, R₂, R₃, R₄ and R₅ are reciprocally independent and each denotes a substituent group selected from the group consisting of a hydrogen atom, a hydroxyl group, an alkoxy group having 1 to 3 carbon atoms, an alkyl group having 1 to 3 carbon atoms, and a hydroxyalkyloxy group having 2 or 3 carbon atoms, wherein two symbols of R₁ and R₂, or two symbols of R₂ and R₃ may be combined together to denote a methylenedioxy group.

2. The process as defined in claim 1, wherein in the formulae (1) to (3), R₂ is a hydroxyl group, R₃ is a methoxy group, and R₁, R₄ and R₅ are a hydrogen atom, and in the formulae (1) and (2), R₂ may be a benzyloxy group.

3. The process as defined in claim 1, wherein in the formulae (1) to (3), R₂ is a methyl group, R₃ is a hydroxyl group, and R₁, R₄ and R₅ are a hydrogen atom, and in the formulae (1) and (2), R₃ may be a benzyloxy group.

4. The process as defined in claim 1, wherein in the formulae (1) to (3), R₂ and R₃ are combined together to denote a methylenedioxy group, and R₁, R₄ and R₅ are a hydrogen atom.

5. The process as defined in claim 1, wherein in the formulae (1) to (3), R₁ is a hydroxyl group, R₃ is a methoxy group, and R₂, R₄ and R₅ are a hydrogen atom, and in the formulae (1) and (2), R₁ may be a benzyloxy group.

6. The process as defined in claim 1, wherein in the formulae (1) to (3), R₃ is a hydroxyl group, and R₁, R₂, R₄ and R₅ are a hydrogen atom, and in the formulae (1) and (2), R₃ may be a benzyloxy group.

7. The process as defined in claim 1, wherein in the formulae (1) to (3), R₂ is a hydroxyl group, R₃ is a methyl group, and R₁, R₄ and R₅ are a hydrogen atom, and in the formulae (1) and (2), R₂ may be a benzyloxy group.

8. The process as defined in claim 1, wherein in the formulae (1) to (3), R₁ and R₃ are a hydroxyl group, and R₂, R₄ and R₅ are a hydrogen atom, and in the formulae (1) and (2), R₁ and/or R₃ may be a benzyloxy group.

9. The process as defined in claim 1, wherein said catalyst is at least one of palladium carbon and platinum carbon.

10. The process as defined in claim 1, wherein a solvent is employed in the reaction for alkylating reductively said aspartame, and said solvent is a methanol or a water-containing methanol.

11. A process for producing a cinnamaldehyde derivative represented by the following general formula (2), which comprises:
subjecting a cinnamic acid derivative represented by the following general formula (4) to a reaction for converting a carboxyl group into a formyl group. 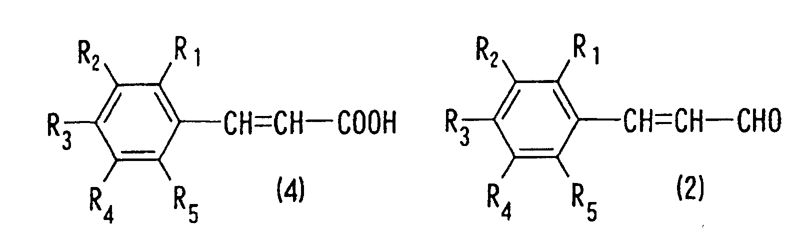
In the formulae (4) and (2), R₁, R₂, R₃, R₄ and R₅ are reciprocally independent and each denotes a substituent group selected from the group consisting of a hydrogen atom, a hydroxyl group, an alkoxy group having 1 to 3 carbon atoms, an alkyl group having 1 to 3 carbon atoms, and a hydroxyalkyloxy group having 2 or 3 carbon atoms, wherein two symbols of R₁ and R₂, or two symbols of R₂ and R₃ may be combined together to denote a methylenedioxy group.

12. The process as defined in claim 11, wherein in the formulae, R₂ is a hydroxyl group, R₃ is a methoxy group, and R₁, R₄ and R₅ are a hydrogen atom.

13. The process as defined in claim 11, wherein said reaction for converting a carboxyl group into a formyl group is a reaction for reducing a carboxyl group by half to a formyl group.

14. A process for producing a hydrocinnamaldehyde derivative represented by the following general formula (1), which comprises:
subjecting a cinnamaldehyde derivative represented by the following general formula (2) to a reaction for reducing a carbon-carbon double bond selectively. 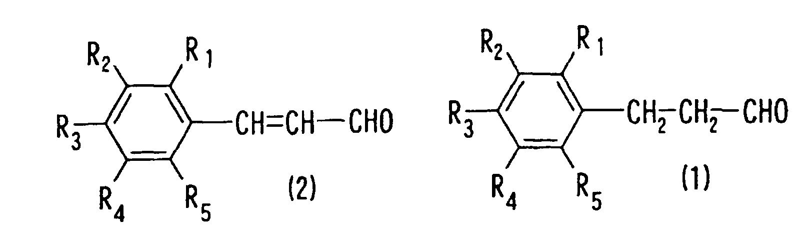
In the formulae (1) and (2), R₁, R₂, R₃, R₄ and R₅ are reciprocally independent and each denotes a substituent group selected from the group consisting of a hydrogen atom, a hydroxyl group, an alkoxy group having 1 to 3 carbon atoms, an alkyl group having 1 to 3 carbon atoms, and a hydroxyalkyloxy group having 2 or 3 carbon atoms, wherein two symbols of R₁ and R₂, or two symbols of R₂ and R₃ may be combined together to denote a methylenedioxy group.

15. The process as defined in claim 14, wherein in the formulae, R₂ is a hydroxyl group, R₃ is a methoxy group, and R₁, R₄ and R₅ are a hydrogen atom.

16. The process as defined in claim 14, wherein said reaction for reducing a carbon-carbon double bond selectively is conducted in the presence of a catalyst for hydrogenation.

17. The process as defined in claim 14, wherein said reaction for reducing a carbon-carbon double bond selectively is conducted in the presence of at least one of palladium, platinum and rhodium based catalysts.

18. A process for producing a hydrocinnamaldehyde derivative, represented by the following general formula (1), which comprises:
subjecting a hydrocinnamic acid derivative represented by the following general formula (5) to a reaction for converting a carboxyl group into a formyl group. 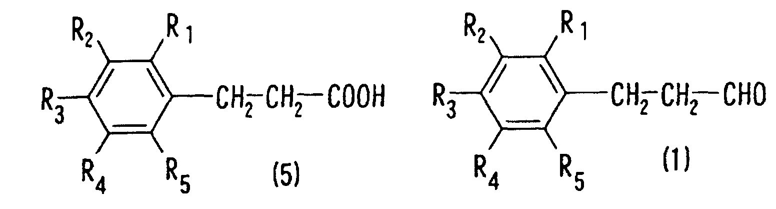
In the formulae (1) and (5), R₁, R₂, R₃, R₄ and R₅ are reciprocally independent and each denotes a substituent group selected from the group consisting of a hydrogen atom, a hydroxyl group, an alkoxy group having 1 to 3 carbon atoms, an alkyl group having 1 to 3 carbon atoms, and a hydroxyalkyloxy group having 2 or 3 carbon atoms, wherein two symbols of R₁ and R₂, or two symbols of R₂ and R₃ may be combined together to denote a methylenedioxy group.

19. The process as defined in claim 18, wherein in the formulae, R₂ is a hydroxyl group, R₃ is a methoxy group, and R₁, R₄ and R₅ are a hydrogen atom.

20. The process as defined in claim 18, wherein said reaction for converting a carboxyl group into a formyl group is a reaction for reducing a carboxyl group by half to a formyl group.

21. A process for producing an aspartyl dipeptide ester derivative represented by the following general formula (3), which comprises:
alkylating reductively an aspartame with the cinnamaldehyde derivative represented by the general formulae (2) obtained in the process as defined in claim 11, and hydrogen in the presence of catalyst: 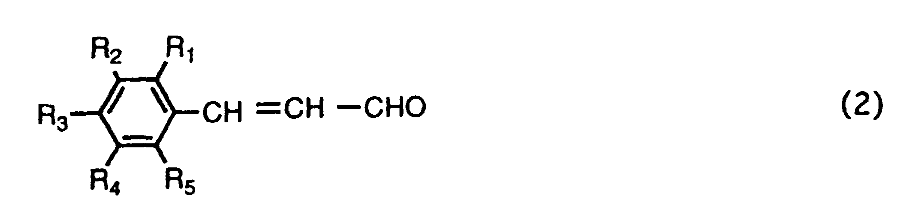 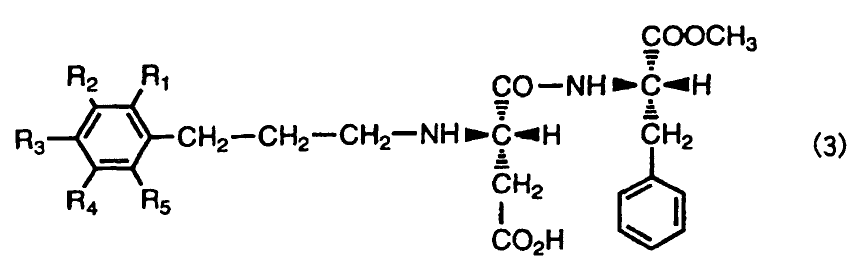
In the formulae (2) and (3), R₁, R₂, R₃, R₄ and R₅ are reciprocally independent and each denotes a substituent group selected from the group consisting of a hydrogen atom, a hydroxyl group, an alkoxy group having 1 to 3 carbon atoms, an alkyl group having 1 to 3 carbon atoms, and a hydroxyalkyloxy group having 2 or 3 carbon atoms, wherein two symbols of R₁ and R₂, or two symbols of R₂ and R₃ may be combined together to denote a methylenedioxy group.

22. The process as defined in claim 21, wherein in the formulae, R₂ is a hydroxyl group, R₃ is a methoxy group, and R₁, R₄ and R₅ are a hydrogen atom.

23. A process for producing an aspartyl dipeptide ester derivative represented by the following general formula (3), which comprises:
alkylating reductively an aspartame with the hydrocinnamaldehyde derivative represented by the general formulae (1) obtained in the process as defined in claim 14 or 18, and hydrogen in the presence of catalyst: 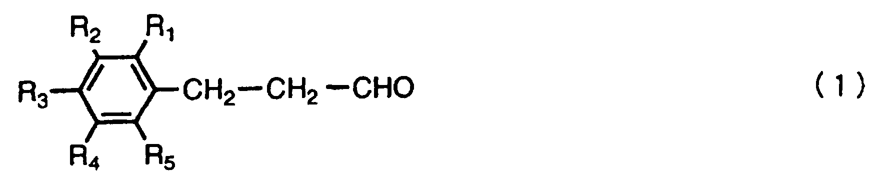 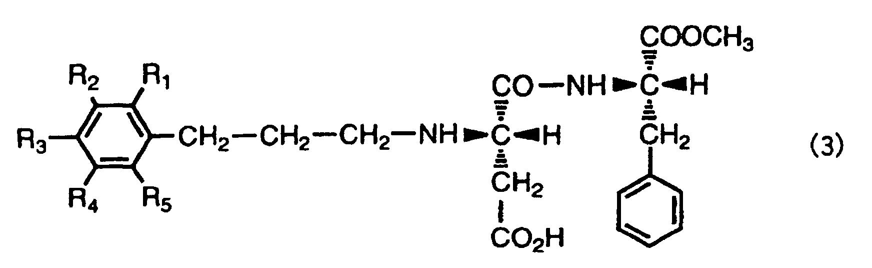
In the formulae (1) and (3), R₁, R₂, R₃, R₄ and R₅ are reciprocally independent and each denotes a substituent group selected from the group consisting of a hydrogen atom, a hydroxyl group, an alkoxy group having 1 to 3 carbon atoms, an alkyl group having 1 to 3 carbon atoms, and a hydroxyalkyloxy group having 2 or 3 carbon atoms, wherein two symbols of R₁ and R₂, or two symbols of R₂ and R₃ may be combined together to denote a methylenedioxy group.

24. The process as defined in claim 23, wherein in the formulae, R₂ is a hydroxyl group, R₃ is a methoxy group, and R₁, R₄ and R₅ are a hydrogen atom.

25. A process for producing a 3-(3-hydroxy-4-methoxyphenyl) propionaldehyde, which comprises:
a. subjecting a 3-hydoroxy-4-methoxycinnamaldehyde to a reaction for reducing a carbon-carbon double bond selectively; or
b. subjecting a 3-(3-hydoroxy-4-methoxyphenyl)propionic acid to a reaction for converting a carboxyl group into a formyl group,
to form a 3-(3-hydroxy-4-methoxyphenyl)propionaldehyde.

26. The process as defined in claim 25, wherein said 3-hydoroxy-4-methoxycinnamaldehyde is obtained in the process for subjecting a 3-hydoroxy-4-methoxycinnamic acid to a reaction for converting a carboxyl group into a formyl group.

27. The process as defined in claim 25 or 26, wherein said reaction for converting a carboxyl group into a formyl group is a reaction for reducing a carboxyl group by half to a formyl group.

28. The process as defined in claim 25, wherein said 3-(3-hydoroxy-4-methoxyphenyl)propionic acid is obtained in the process for subjecting a 3-hydoroxy-4-methoxycinnamic acid to a reaction for reducing a carbon-carbon double bond selectively.

29. The process as defined in claim 25 or 28, wherein said reaction for reducing a carbon-carbon double bond selectively is conducted in the presence of a catalyst for hydrogenation.

30. The process as defined in claim 29, wherein said catalyst is at least one of palladium, platinum and rhodium based catalysts.

31. A process for producing a N-[N-[3-(3-hydroxy-4-methoxyphenyl) propyl]-L- α -aspartyl]-L-phenylalanine 1-methyl ester, which comprises:
subjecting further a 3-(3-hydroxy-4-methoxyphenyl) propionaldehyde obtained in the process as defined in claim 25 to 30, to a reductive alkylation reaction with an aspartame.

32. A novel compound of 3-(3-hydroxy-4-methoxyphenyl) propionaldehyde.

33. A 3-(3-hydroxy-4-methoxyphenyl) propionaldehyde obtained in the process as defined in any one of claims 25 to 31.
